(19) Europäisches Patentamt
European Patent Office
Office européen des brevets

(11) Veröffentlichungsnummer: **0 017 771**
**B1**

(12) # EUROPÄISCHE PATENTSCHRIFT

(45) Veröffentlichungstag der Patentschrift: **23.09.81**

(51) Int. Cl.³: **C 07 C 65/11,** C 07 C 51/47, C 07 C 51/02 // C07C39/14, C07C37/68

(21) Anmeldenummer: **80101391.3**

(22) Anmeldetag: **17.03.80**

(54) **Verfahren zur Gewinnung von 2-Hydroxynaphthalin-3-carbonsäure aus den Umsetzungsgemischen der Alkalisalze des 2-Hydroxynaphthalins mit Kohlendioxid.**

(30) Priorität: **24.03.79 DE 2911667**

(43) Veröffentlichungstag der Anmeldung: **29.10.80 Patentblatt 80/22**

(45) Bekanntmachung des Hinweises auf die Patenterteilung: **23.09.81 Patentblatt 81/38**

(84) Benannte Vertragsstaaten: **CH DE FR GB IT**

(56) Entgegenhaltungen: **DE-A-2 426 852** **DE-B-1 643 541**

(73) Patentinhaber: **HOECHST AKTIENGESELLSCHAFT, Postfach 80 03 20, D-6230 Frankfurt/Main 80 (DE)**

(72) Erfinder: **Volk, Heinrich, Dr., Am weissen Stein 1, D-6368 Bad Vilbel (DE)**
Erfinder: **Papenfuhs, Theodor, Dr., Heinrich-Bleicher-Strasse 40, D-6000 Frankfurt am Main 50 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 017 771 B1

BUNDESDRUCKEREI BERLIN

# Verfahren zur Gewinnung von 2-Hydroxynaphthalin-3-carbonsäure aus den Umsetzungsgemischen der Alkalisalze des 2-Hydroxynaphthalins mit Kohlendioxid

Die vorliegende Erfindung liegt auf dem Gebiet der Herstellung von Zwischenprodukten, insbesondere des bekannten Vorproduktes für wasserunlösliche Azofarbstoffe, der 2-Hydroxynaphthalin-3-carbonsäure.

Bei der Carboxylierung des Natriumsalzes des 2-Hydroxynaphthalins unter Druck und bei Temperaturen über 210°C entstehen neben dem Natriumsalz der 2-Hydroxynaphthalin-3-carbonsäure und freiem 2-Hydroxynaphthalin beträchtliche Mengen an harzartigen Nebenprodukten, die bei den bekannten technischen Verfahren auf sehr aufwendige Weise von der gewünschten Naphtholcarbonsäure abgetrennt werden (vgl. FIAT Final Report Nr. 1308). Diese höhermolekularen, in ihrer Konstitution meist nicht bekannten Verunreinigungen lassen sich in zwei Gruppen einteilen, und zwar in die der alkaliunlöslichen Harze und in die der alkalilöslichen Harze. Die alkaliunlöslichen Harze enthalten vermutlich Einheiten mit Xanthen- oder Ketonstruktur, und die alkalilöslichen Harze vermögen wegen ihres Gehaltes an Hydroxy- und/oder Carboxygruppen in Wasser mehr oder minder lösliche Salze zu bilden. Während die alkaliunlöslichen Harze aus der mit Wasser vermischten heißen, alkalischen Reaktionsmischung der Carboxylierungsreaktion leicht mit gängigen Methoden, wie durch Filter, Separatoren oder ähnliches, von der wäßrig-alkalischen Lösung der 2-Hydroxynaphthalin-3-carbonsäure abgetrennt werden können, ist es bis jetzt nur technisch unbefriedigend möglich, die wasserlöslichen Harze, vor allem deren stärker sauren Komponenten mit Carboxygruppen, aus dem Reaktionsgemisch zu entfernen.

So geht man beispielsweise in der technisch praktizierten Aufarbeitung der wasserverdünnten Carboxylierungsschmelze in der Weise vor, daß man diese zuerst auf einen pH-Wert von etwa 6 bis 8 neutralisiert, wodurch eventuell noch vorliegendes Natriumsalz des 2-Hydroxynaphthalins in freies 2-Hydroxynaphthalin überführt wird, das sich bei den bevorzugten Temperaturen von 80 bis 95°C in Wasser löst. Bei dieser Einstellung des pH-Wertes von etwa 6 bis 8 fallen die durch phenolische Gruppen nur schwach sauren Anteile der alkalilöslichen Harze aus und können zusammen mit den bereits bei der Vermischung der Carboxylierungsschmelze mit Wasser sich abscheidenden alkaliunlöslichen Harzen in üblicher Weise abgetrennt werden. Aus der verbleibenden neutralisierten heißen wäßrigen Lösung des Mononatriumsalzes der 2-Hydroxynaphthalin-3-carbonsäure und 2-Hydroxynaphthalins scheidet sich sodann beim Abkühlen auf 20 bis 30°C das gelöste 2-Hydroxynaphthalin aus, das anschließend beispielsweise durch Filtration von der wäßrigen Lösung des Verfahrensproduktes abgetrennt werden kann. Die in der wäßrigen Lösung als Natriumsalze weiterhin enthaltenen stärker sauren höhermolekularen Harze ließen sich bisher von der 2-Hydroxynaphthalin-3-carbonsäure nicht abtrennen, da sie bei der Isolierung der 2-Hydroxynaphthalin-3-carbonsäure durch saure Fällung bei erhöhter Temperatur (80 bis 95°C) mit ausfielen und somit zu einem durch Harz verunreinigten Produkt führten, was natürlich bei der Weiterverwendung dieses Endproduktes, beispielsweise für die Herstellung von Arylamiden der 2-Hydroxynaphthalin-3-carbonsäure, die als Kupplungskomponenten in der Eisfarbentechnik und als Vorprodukte in der Farbpigmentherstellung eine überragende technische Rolle spielen, erhebliche Qualitätsprobleme mit sich bringt.

Es bestand also ein dringendes Bedürfnis, eine Methode zu finden, diese stärker sauren Harze von der gewünschten 2-Hydroxynaphthalin-3-carbonsäure abzutrennen und eine Separierungsmethode zu finden, die sich problemlos in die übliche Aufarbeitung des Carboxylierungsgemisches zur 2-Hydroxynaphthalin-3-carbonsäure eingliedert, insbesondere um zusätzliche Investitions- und Arbeitskosten so weit wie möglich zu vermeiden.

Mit der vorliegenden Erfindung wurde diese Aufgabe gelöst.

Es wurde nämlich ein Verfahren zur Gewinnung von 2-Hydroxynaphthalin-3-carbonsäure gefunden, bei welchem man diese 2-Hydroxynaphthalin-3-carbonsäure aus der mit Wasser verdünnten Carboxylierungschmelze, die durch Umsetzung des Natriumsalzes des 2-Hydroxynaphthalins mit Kohlendioxid erhalten wurde, in einfacher Weise und als reine Verbindung erhält, wenn man erfindungskennzeichnend die alkalilöslichen sauren Harze mittels einer kationischen oberflächenaktiven Verbindung, die vorzugsweise ein quartäres Stickstoffatom enthält, während des Aufarbeitungsprozesses des Carboxylierungsproduktes abtrennt.

Bei der Aufarbeitung der wäßrigen Lösung der Carboxylierungsschmelze kann die erfindungsgemäß verwendete kationische oberflächenaktive Verbindung vor dem Aufarbeitungsschritt, mit welchem die 2-Hydroxynaphthalin-3-carbonsäure sauer gefällt wird, besonders vorteilhaft jedoch nach der Neutralisation der alkalischen, in Wasser gelösten Carboxylierungsschmelze zugegeben werden (unter Neutralisation wird hier nicht nur die Einstellung von einem pH-Wert von etwa 7, sondern auch die Einstellung auf einen schwach sauren Bereich bis 3,5 verstanden).

Die erfindungsgemäß verwendbaren kationischen oberflächenaktiven Verbindungen ergeben nämlich überraschenderweise mit den stark sauren Harzen wasserunlösliche Fällungen, die sich, abhängig von den gewählten Fälltemperaturen und abhängig von der Konstitution der oberflächenaktiven Verbindung, in fester oder flüssiger Form bilden können. Hierbei ist über die Natur dieser Fällungen der stark sauren Harze nichts bekannt. Die ausfallenden wasserunlöslichen

Verbindungen können Salze aus einer Verbindung dieser kationischen oberflächenaktiven Verbindung und den sauren Harzen sein, es können aber auch anders geartete Bindungsverhältnisse, wie beispielsweise Aggregate oder Molekülverbindungen, vermutet werden.

Erfindungsgemäß verwendbare kationische oberflächenaktive Verbindungen sind vor allem quaternäre Ammoniumverbindungen entsprechend der allgemeinen Formel (1)

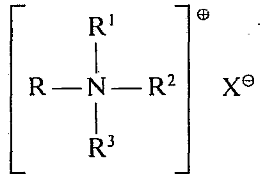

(1)

in welcher R, $R^1$, $R^2$ und $R^3$ zueinander gleich oder voneinander verschieden sein können und jedes einen aliphatischen Rest, einen Aralkylrest oder einen Arylrest bedeutet und wobei die Summe der C-Atome der Substituenten R, $R^1$, $R^2$ und $R^3$ mindestens 8 beträgt; zwei oder drei der aliphatischen Reste können auch gemeinsam mit dem Stickstoffatom einen heterocyclischen, gegebenenfalls Doppelbindungen enthaltenden Ring bilden, an dem ein Benzolkern ankondensiert sein kann, wie beispielsweise einen Pyridin-, Morpholin-, Imidazolin-, Benzimidazolin-, Imidazol-, Benzimidazol- oder Oxazolring. Der Formelrest $X^{\ominus}$ stellt das Äquivalent einer anorganischen oder organischen Säure dar.

Unter dem Betriff »aliphatischer Rest« für die Formelreste R, $R^1$, $R^2$ und $R^3$ werden allgemein aliphatische geradkettige oder verzweigte Kohlenwasserstoffreste von 1 bis 30 C-Atomen verstanden, wobei diese eine oder mehrere, wie 1, 2 oder 3 Doppelbindungen, und/oder ein oder mehrere, wie 1, 2 oder 3 Heteroatome, wie beispielsweise Gruppen der Formeln —O—, —S—, —NH— oder substituierte Aminogruppen oder quaternierte Stickstoffatome, und/oder andere Gruppen, wie beispielsweise Carbonsäureamidgruppen, in der aliphatischen Kette gebunden enthalten können und/oder die aliphatischen Reste über ein Sauerstoffatom an das quartäre Stickstoffatom gebunden sein können. Diese aliphatischen Reste R, $R^1$, $R^2$ und $R^3$ können weiterhin nichtionogene Substituenten, wie beispielsweise Hydroxy-, Alkyloxy- oder Polyglykoläthergruppen enthalten, wobei diese Substituenten bevorzugt aus 1 bis 30 C-Atomen bestehen. Weiterhin werden unter »aliphatische Reste« hydroaromatische carbocyclische und cycloaliphatische Reste von jeweils 4 bis 8 Ring-C-Atomen verstanden, wobei diese cyclischen Reste aliphatische Seitenketten von 1 bis 12 C-Atomen oder Halogenatome, wie Chloratome, tragen können.

Aralkylreste der Formelreste R, $R^1$, $R^2$ und $R^3$ sind bevorzugt aliphatische Reste von 1 bis 30 C-Atomen, bevorzugt von 1 bis 12 C-Atomen, die durch Naphthyl- und/oder Phenylreste substituiert sein können, wobei diese Arylreste noch andere Substituenten, wie beispielsweise Hydroxygruppen, niedere Alkylgruppen, niedere Alkoxygruppen und/oder Halogenatome, wie Chloratome, enthalten können. Aralkylreste sind beispielsweise Benzyl-, Phenylnonyl- oder Phenyldodecyl-Reste. Arylreste sind aromatische carbocyclische Reste, bevorzugt Phenyl- und Naphthylreste, die durch Hydroxy, niederes Alkoxy, niederes Alkyl, Halogen, wie Chlor, Carbonamid und/oder Sulfonamid substituiert sein können.

Das Anion $X^{\ominus}$ ist beispielsweise das einer Halogenwasserstoffsäure, wie das Chlorid- oder Bromidanion, oder das einer anderen anorganischen Säure, wie beispielsweise das Sulfat- oder Hydrogensulfatanion oder ein Anion der Phosphorsäure oder der Borsäure, oder das Anion einer organischen Carbonsäure, wie beispielsweise das Anion der Essigsäure, der Ameisensäure, Oxalsäure, Milchsäure, Weinsäure, Gluconsäure, Zitronensäure oder Benzoesäure, oder der Rest einer organischen Sulfosäure, wie beispielsweise das Anion der Methansulfosäure oder Benzolsulfosäure.

Erfindungsgemäß verwendbare kationische oberflächenaktive Verbindungen sind aber auch oberflächenaktive Sulfonium- und Phosphoniumverbindungen der allgemeinen Formel (2) bzw. (3)

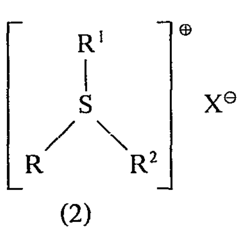

(2)

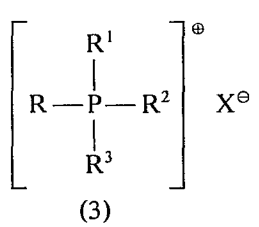

(3)

wobei in Formel (2) die Reste R, $R^1$, $R^2$ und $X^{\ominus}$ die für Formel (1) genannten Bedeutungen haben, die Summe der C-Atome von R, $R^1$ und $R^2$ jedoch mindestens 6 ist, und zwei oder drei der aliphatischen Reste gemeinsam mit dem Schwefelatom einen heterocyclischen, gegebenenfalls Doppelbindungen enthaltenden Ring bilden können, und wobei in Formel (3) die Reste R, $R^1$, $R^2$, $R^3$ und $X^{\ominus}$ die für Formel (1) genannten Bedeutungen haben und die Summe der C-Atome von R, $R^1$, $R^2$ und $R^3$ mindestens 8 ist und zwei oder drei der aliphatischen Reste gemeinsam mit dem Phosphoratom einen heterocyclischen, gegebenenfalls Doppelbindungen enthaltenden Ring bilden können.

Bevorzugt sind Verbindungen der allgemeinen Formel (1), in welcher R einen Alkylrest von 8 bis 20 C-Atomen bedeutet, $R^1$ für einen Alkylrest von 1 bis 20 C-Atomen oder für einen Di- bis Hexaäthylenglykoläther-Rest oder für einen Hydroxyalkylrest mit 2 bis 6 C-Atomen oder für einen Phenylalkylrest mit 4 bis 12 C-Atomen im Alkylrest steht, $R^2$ einen Alkylrest von 1 bis 8 C-Atomen bedeutet oder ein Hydroxyalkylrest von 2 bis 6 C-Atomen ist und $R^3$ ein Alkylrest von 1 bis 8 C-Atomen oder den Benzylrest darstellt.

Erfindungsgemäß verwendbare quartäre Ammoniumverbindungen der allgemeinen Formel (1) sind beispielsweise:

Dodecyl-dimethyl-benzyl-ammoniumchlorid,
Oleyl-trimethyl-ammoniumchlorid,
Distearyl-dimethyl-ammoniumchlorid,
Lauryl-dimethyl-hydroxyäthyl-ammoniumchlorid,
Dodecyl-di-(hydroxyäthyl)-methyl-ammoniumchlorid,
Dodecyl-di-methyl-vinyl-ammoniumchlorid,
Dodecyl-methyl-morpholiniumchlorid,
Lauryl-pyridiniumchlorid,
Hexadecyl-N,N'-dimethyl-benzimidazoliniumsulfat,
Dodecyl-di-(triäthylen-glykoläther)-benzyl-ammoniumchlorid,
Phenylnonyl-dimethyl-benzyl-ammoniumchlorid,
Oleyl-di-(hydroxyäthyl)-äthylenglykoläther-ammoniumchlorid,
Oleyl-dimethyl-hydroxyäthyl-ammoniumchlorid,
Cocos-di-(triäthylenglykoläther)-benzyl-ammoniumchlorid,
Cocos-dimethyl-benzyl-ammoniumchlorid,
Distearyl-dimethyl-ammoniumchlorid,
Trioctyl-methyl-ammoniumchlorid,
Cocos-dimethyl-hydroxypropyl-ammoniumchlorid,
p-(iso-Butyl)-phenoxyäthoxyäthyl-dimethyl-benzyl-ammoniumchlorid,
Oleyl-methyl-imidazoliniumchlorid,
Hexadecyl-N,N'-dimethyl-benzimidazoliumsulfat,
Oleyl-methyl-imidazoliumchlorid,
Dehydro-abietyl-dimethyl-benzyl-ammoniumchlorid,
bevorzugt beispielsweise Dodecyl-dimethyl-ammoniumchlorid,
Stearyl-dimethyl-benzyl-ammoniumchlorid,
Cocos-dimethyl-2,4-dichlorbenzyl-ammoniumchlorid,
Trioctyl-methyl-ammoniumchlorid,
Cetyl-pyridiniumchlorid,
Tetra-(n-octyl)-phosphoniumchlorid,
Tetrabutyl-ammoniumhydrogensulfat,
($C_{12}$—$C_{16}$-Alkyl)-dimethyl-benzyl-ammoniumchlorid,
Tetrabutyl-phosphoniumbromid,
insbesondere jedoch Cocos-benzyl-dimethyl-ammoniumchlorid und
Benzyl-trimethyl-ammoniumchlorid.
(Unter dem Rest »Cocos« wird ein aliphatischer Rest aus einem Gemisch von Alkyl-, Alkenyl- und Alk-dienyl-Resten, jeweils von 8 bis 18 C-Atomen, verstanden.)

Eine bevorzugte Ausführungsform des Aufarbeitungsverfahrens der Carboxylierungsschmelze besteht darin, daß man das Carboxylierungsgemisch mit Wasser bei einer Temperatur von mindestens 80°C verdünnt bzw. darin löst, diese Lösung mit einer wäßrigen Mineralsäure, wie vorzugsweise Salzsäure oder Schwefelsäure, neutralisiert und bevorzugt auf einen pH-Wert von 3,5 bis 6,8, vorzugsweise von 4 bis 6,5, einstellt, unlösliche Teile davon abtrennt, bspw. durch Filtration, das Filtrat auf eine Temperatur von etwa 10 bis 30°C, vorzugsweise 15 bis 25°C, abkühlt, das abgeschiedene 2-Hydroxynaphthalin abtrennt, dessen Filtrat mit wäßriger Mineralsäure, vorzugsweise Salz- oder Schwefelsäure, auf einen pH-Wert von 0,5 bis 2,5, vorzugsweise von 1 bis 2, einstellt und die abgeschiedene 2-Hydroxynaphthalin-3-carbonsäure isoliert, wobei man die erfindungsgemäß verwendbare kationische oberflächenaktive Verbindung nach der Abtrennung des abgeschiedenen 2-Hydroxynaphthalins und vor Einstellung des pH-Wertes auf 0,5 bis 2,5 zugibt und das gefällte Harz abtrennt oder erfindungsgemäß besonders vorteilhaft die kationische oberflächenaktive Verbindung direkt nach der Neutralisation der in Wasser gelösten Carboxylierungsschmelze in den Aufarbeitungsprozeß eingibt.

Die erstgenannte Verfahrensvariante der Zugabe der kationischen oberflächenaktiven Verbindung vor der sauren Fällung der 2-Hydroxynaphthalin-3-carbonsäure erfordert einen zusätzlichen Schritt zur Abtrennung der mit der erfindungsgemäß verwendbaren Verbindung erhaltenen Fällung des Harzes; diese Abtrennung erfolgt beispielsweise, insbesondere abhängig von der Art der verwendeten kationischen oberflächenaktiven Verbindung, mittels eines Separators oder einer Filterpresse. Die

zweite Verfahrensvariante hat gegenüber der ersten den Vorteil, daß die Abtrennung der mit der oberflächenaktiven Verbindung erhaltenen Harzfällung im gleichen Schritt erfolgt wie die Abtrennung des alkaliunlöslichen Harzes, so bei der Filtration der mit einer wäßrigen Mineralsäure neutral eingestellten, insbesondere auf einen pH-Wert von 3,5 bis 6,8 eingestellten Lösung der Carboxylierungsschmelze; bei dieser zweiten, bevorzugten Alternative, bei welcher das alkalilösliche, saure Harz gefällt und zusammen mit dem alkaliunlöslichen Harz abgetrennt wird, ist kein zusätzlicher Abtrennungsschritt erforderlich, sondern das übliche Aufarbeitungsverfahren von technischen Carboxylierungsgemischen kann unverändert beibehalten werden.

Die besonders vorteilhafte Durchführung des erfindungsgemäßen Verfahrens zur Gewinnung einer reinen 2-Hydroxynaphthalin-3-carbonsäure aus den Umsetzungsgemischen des Natriumsalzes des 2-Hydroxynaphthalins mit Kohlendioxid besteht deshalb darin, daß man die mit Wasser bei einer Temperatur von mindestens 80°C verdünnte und darin gelöste Carboxylierungsschmelze mit einer wäßrigen Mineralsäure neutralisiert, vorzugsweise auf einen pH-Wert von 4 bis 6,5 einstellt, eine kationische oberflächenaktive Verbindung hinzugibt, die unlöslichen Anteile von der Lösung abtrennt, die geklärte Lösung auf eine Temperatur von 10 bis 30°C abkühlt, das abgeschiedene 2-Hydroxynaphthalin abtrennt, das Filtrat mit wäßriger Mineralsäure auf einen pH-Wert von 0,5 bis 2,5 einstellt und daraus die abgeschiedene 2-Hydroxynaphthalin-3-carbonsäure isoliert.

Die Fällung der alkalilöslichen sauren Harze aus den Carboxylierungsschmelzen mittels der kationischen oberflächenaktiven Verbindung erfolgt beim Zusatz der oberflächenaktiven Verbindung zu der wäßrigen, »neutralisierten« Carboxylierungslösung. In der Regel werden die kationischen oberflächenaktiven Verbindungen in einer Menge von 0,1 bis 5,0 Gew.-%, vorzugsweise von 0,5 bis 3,0 Gew.-%, jeweils bezogen auf die in der Lösung enthaltene 2-Hydroxynaphthalin-3-carbonsäure, eingesetzt. Die Fällung der alkalilöslichen Harze durch diese erfindungsgemäß verwendeten kationischen oberflächenaktiven Verbindungen ist quantitativ.

Es war überraschend, daß mit den erfindungsgemäß verwendbaren kationischen oberflächenaktiven Verbindungen eine Abtrennung der alkalilöslichen sauren Harze aus den Carboxylierungsschmelzen durch Fällung aus den wäßrigen Lösungen möglich war. Es ist nämlich aus der deutschen Auslegeschrift 1 643 541 bekannt, daß man kationische oberflächenaktive Verbindungen entsprechend der allgemeinen Formel (1) dazu verwendet, um bei Phenolat-Carboxylierungen nach dem Vermischen mit Wasser und anschließendem Ansäuern zur Fällung der gewünschten Hydroxybenzoesäure die in diesen Carboxylierungsproduktgemischen enthaltenen Verunreinigungen in Lösung zu halten und zu vermeiden, daß diese Verunreinigungen zusammen mit der gewünschten Hydroxybenzoesäure ausfallen. Es war deshalb nicht zu erwarten, daß die kationischen oberflächenaktiven Verbindungen, die in dem vorliegenden erfindungsgemäßen Verfahren eingesetzt werden, zur Abtrennung der alkalilöslichen Harze einsetzbar sind.

Die nach dem erfindungsgemäßen Verfahren gewinnbare 2-Hydroxynaphthalin-3-carbonsäure ist von überragender Qualität und Reinheit; die erfindungsgemäß gewonnene 2-Hydroxynaphthalin-3-carbonsäure läßt sich deshalb problemlos zu reinen Folgeprodukten, wie obenerwähnt, weiterverarbeiten, die wiederum als sehr reine Verbindungen zur Herstellung von wertvollen Farbpigmenten oder als Kupplungskomponenten zur Herstellung reiner Färbungen gemäß der Eisfarbentechnik dienen können.

Die nachstehenden Beispiele dienen zur Erläuterung der Erfindung; die dort gemachten Prozentangaben beziehen sich auf Gewichtsprozente, sofern nicht anders vermerkt.

### Beispiel 1

2500 ml einer wäßrigen Lösung aus einer Carboxylierungsschmelze aus einer Carboxylierungsreaktion des Natriumsalzes des 2-Hydroxynaphthalins mit Kohlendioxid, die im Liter Lösung beispielsweise 40 g 2-Hydroxynaphthalin-3-carbonsäure enthält, wird bei 80° C mit einer 30%igen wäßrigen Salzsäure auf einen pH-Wert von 6,0 eingestellt. Das alkaliunlösliche Harz läßt man im Verlauf von 5 Minuten sich absetzen und filtriert es sodann ab. Das rötlich-braune Filtrat wird auf eine Temperatur von 20°C abgekühlt, das ausgefallene 2-Hydroxynaphthalin abfiltriert.

Dieses Filtrat wird auf 60° C erwärmt. Mit 78%iger wäßriger Schwefelsäure wird ein pH-Wert von 5,0 eingestellt. Man gibt 1 g Cocos-benzyl-dimethyl-ammoniumchlorid hinzu und rührt die Lösung 5 Minuten lang bei 60° C. Danach läßt man das gefällte Harz im Verlaufe von 5 Minuten sich absetzen; es wird abfiltriert, die klare Lösung auf 80° C erwärmt und ein pH-Wert von 2,0 mittels 78%iger wäßriger Schwefelsäure eingestellt. Die ausgefallene 2-Hydroxynaphthalin-3-carbonsäure wird bei 80°C abgesaugt und bei 80° C bis zur Gewichtskonstanz getrocknet. Man erhält 98,5 g einer hochreinen 2-Hydroxynaphthalin-3-carbonsäure.

### Beispiel 2

Man verfährt zur Aufarbeitung einer Carboxylierungsschmelze und zur Abtrennung der alkalilöslichen Harze daraus wie in Beispiel 1 beschrieben, setzt jedoch anstelle des dort verwendeten

kationischen oberflächenaktiven Mittels die gleiche Menge an einer anderen erfindungsgemäß verwendbaren kationischen oberflächenaktiven Verbindung ein, so beispielsweise Dodecyl-dimethyl-ammoniumchlorid oder Stearyl-dimethyl-benzyl-ammoniumchlorid oder Cocos-dimethyl-2,4-dichlorbenzyl-ammoniumchlorid oder Benzyl-trimethyl-ammoniumchlorid oder Trioctyl-methyl-ammoniumchlorid oder Cetyl-pyridiniumchlorid oder Tetra-(n-octyl)-phosphoniumchlorid oder Tetrabutyl-ammoniumhydrogensulfat. Man erhält in gleicher Weise wie in Beispiel 1 die 2-Hydroxynaphthalin-3-carbonsäure in der angegebenen hohen Ausbeute und als hochreiche Substanz.

Beispiel 3

2500 ml der in Beispiel 1 angegebenen Lösung aus der Carboxylierungsschmelze mit einem Gehalt von 40 g 2-Hydroxynaphthalin-3-carbonsäure im Liter werden bei 80° C mit einer 30%igen wäßrigen Salzsäure auf einen pH-Wert von 6,0 eingestellt. Anschließend gibt man 3 g Cocos-benzyl-dimethyl-ammoniumchlorid in Form einer 50%igen wäßrigen Lösung hinzu, rührt das Ganze fünf Minuten lang bei 80° C, läßt daraufhin 5 Minuten lang absitzen und filtriert das ausgefällte Harz zusammen mit dem alkaliunlöslichen Harz ab. Das helle Filtrat wird nun auf eine Temperatur von 20° C abgekühlt und das ausgefallene 2-Hydroxynaphthalin abfiltriert. Das Filtrat erwärmt man sodann auf 80° C und stellt es mit einer 78%igen wäßrigen Schwefelsäure auf einen pH-Wert von 2,0 ein. Die hiernach ausgefallene 2-Hydroxynaphthalin-3-carbonsäure wird bei einer Temperatur von 80° C abgesaugt und bei 80° C bis zur Gewichtskonstanz getrocknet. Man erhält sie in einer Ausbeute von 98,0 g in hoher Reinheit, die der der 2-Hydroxynaphthalin-3-carbonsäure von Beispiel 1 entspricht.

Beispiel 4

2500 ml einer in Wasser gelösten Carboxylierungsschmelze mit einem Gehalt von 60 g 2-Hydroxynaphthalin-3-carbonsäure im Liter werden bei 80° C mit einer wäßrigen 30%igen Salzsäure auf einen pH-Wert von 6,0 eingestellt. Man gibt 6,5 g Cocos-benzyl-dimethyl-ammoniumchlorid hinzu, rührt die Lösung 5 Minuten lang bei 80° C, läßt 5 Minuten lang absitzen und filtriert das Harz ab. Die erhaltene klare Lösung wird auf 20° C abgekühlt, das ausgefallene 2-Hydroxynaphthalin abfiltriert. Die weitere Aufarbeitung des Filtrates erfolgt gemäß den Angaben des Beispiels 1. Man erhält 147,6 g einer hochreinen 2-Hydroxynaphthalin-3-carbonsäure.

Beispiel 5

2500 ml der in Beispiel 1 angegebenen Ausgangslösung der Carboxylierungsschmelze werden bei 80° C mit 78%iger wäßriger Schwefelsäure auf einen pH-Wert von 4,5 eingestellt. Man gibt zu ihr 0,5 g Benzyl-trimethyl-ammoniumchlorid hinzu, rührt das Ganze 5 Minuten lang bei 80° C, läßt während 5 Minuten absitzen und filtriert sodann das Harz ab. Das Filtrat wird sodann auf 20° C abgekühlt und das ausgefallene 2-Hydroxynaphthalin abfiltriert. Die weitere Aufarbeitung erfolgt in der im Beispiel 1 beschriebenen Weise. Man erhält die 2-Hydroxynaphthalin-3-carbonsäure in hoher Reinheit mit einer Ausbeute von 98,2 g.

Beispiel 6

2500 ml der in Beispiel 1 angegebenen Ausgangslösung der Carboxylierungsschmelze werden bei 80° C mit einer 30%igen wäßrigen Salzsäure auf einen pH-Wert von 6,0 eingestellt. Man filtriert diese Lösung bei 80° C und kühlt sie sodann auf 20° C ab. Das ausgefallene 2-Hydroxynaphthalin wird abfiltriert, das Filtrat bei 20° C mit 4,0 g Cocos-benzyl-dimethyl-ammoniumchlorid versetzt. Man rührt 15 Minuten bei dieser Temperatur nach, filtriert das abgeschiedene Harz ab, erwärmt die klare Lösung auf eine Temperatur von 80° C und arbeitet im übrigen gemäß den Angaben des Beispiels 1 auf. Man erhält die hochreine 2-Hydroxynaphthalin-3-carbonsäure in einer Ausbeute von 98,3 g.

Beispiel 7

Zur Aufarbeitung einer Carboxylierungsschmelze aus dem Natriumsalz des 2-Hydroxynaphthalins mit Kohlendioxid und zur Ausfällung des alkalilöslichen Harzes verfährt man gemäß der

Verfahrensweise des Beispiels 1, setzt jedoch anstelle des dort verwendeten Cocos-benzyl-dimethylammoniumchlorids als kationisches oberflächenaktives Mittel die gleiche Menge an einem anderen erfindungsgemäß verwendbaren kationischen oberflächenaktiven Mittel ein, so beispielsweise ($C_{12}-C_{16}$-Alkyl)-dimethyl-benzyl-ammoniumchlorid oder Cocos-dimethyl-2,4-di-chlorbenzyl-ammoniumchlorid oder Benzyl-trimethyl-ammoniumchlorid oder Trioctyl-methyl-ammoniumchlorid oder Tetrabutyl-ammoniumhydrogensulfat oder Tetrabutyl-phosphoniumbromid ein. Man erhält in gleicher Weise wie in Beispiel 6 die 2-Hydroxynaphthalin-3-carbonsäure als hochreine Verbindung mit einer Ausbeute von 98,0 bis 98,5 g.

**Patentansprüche**

1. Ein Verfahren zur Gewinnung der 2-Hydroxynaphthalin-3-carbonsäure aus dem Umsetzungsprodukt des Natriumsalzes des 2-Hydroxynaphthalins mit Kohlendioxid, bei welchem man die Carboxylierungsschmelze mit Wasser verdünnt und darin löst und die darin befindlichen Produkte auftrennt und abtrennt, dadurch gekennzeichnet, daß man während des Aufarbeitungsprozesses des Carboxylierungsproduktes die alkalilöslichen sauren Harze mittels einer kationischen oberflächenaktiven Verbindung abtrennt.

2. Verfahren nach Anspruch 1, dadurch gekennzeichnet, daß die verwendete kationische oberflächenaktive Verbindung ein quartäres Stickstoffatom enthält.

3. Verfahren nach Anspruch 1, dadurch gekennzeichnet, daß die verwendete kationische oberflächenaktive Verbindung eine Verbindung der allgemeinen Formel (1)

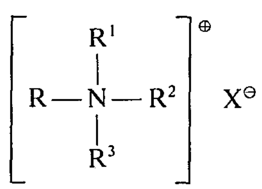
(1)

ist, in welcher R, $R^1$, $R^2$ und $R^3$ zueinander gleich oder voneinander verschieden sind und jedes einen gegebenenfalls substituierten aliphatischen Rest, einen Aralkylrest oder einen Arylrest bedeutet, wobei zwei oder drei der aliphatischen Reste gemeinsam mit dem Stickstoffatom einen heterocyclischen Ring bilden können, mit der Maßgabe, daß die Summe der C-Atome der Substituenten R, $R^1$, $R^2$ und $R^3$ mindestens 8 ist, und in welcher $X^{\ominus}$ das Äquivalent einer anorganischen oder organischen Säure darstellt.

4. Verfahren nach Anspruch 3, dadurch gekennzeichnet, daß in der verwendeten kationischen oberflächenaktiven Verbindung der allgemeinen Formel (1) R einen Alkylrest von 8 bis 20 C-Atomen bedeutet, $R^1$ für einen Alkylrest von 1 bis 20 C-Atomen oder für einen Di- bis Hexaäthylenglykoläther-Rest oder für einen Hydroxyalkylrest mit 2 bis 6 C-Atomen oder für einen Phenylalkylrest mit 4 bis 12 C-Atomen im Alkylrest steht, $R^2$ einen Alkylrest von 1 bis 8 C-Atomen bedeutet oder ein Hydroxyalkylrest von 2 bis 6 C-Atomen ist und $R^3$ einen Alkylrest von 1 bis 8 C-Atomen oder den Benzylrest darstellt.

5. Verfahren nach Anspruch 1, dadurch gekennzeichnet, daß die verwendete kationische oberflächenaktive Verbindung Cocos-benzyl-dimethyl-ammoniumchlorid ist.

6. Verfahren nach einem der Ansprüche 1 bis 5, dadurch gekennzeichnet, daß man die oberflächenaktive kationische Verbindung in einer Menge von 0,1 bis 5 Gew.-%, bezogen auf die im Carboxylierungsgemisch enthaltene 2-Hydroxynaphthalin-3-carbonsäure, verwendet.

7. Verfahren nach einem der Ansprüche 1 bis 5, dadurch gekennzeichnet, daß man die oberflächenaktive kationische Verbindung in einer Menge von 0,5 bis 3,0 Gew.-%, bezogen auf die im Carboxylierungsgemisch enthaltene 2-Hydroxynaphthalin-3-carbonsäure, verwendet.

**Claims**

1. A process for extracting 2-hydroxynaphthalene-3-carboxylic acid from the reaction product of the sodium salt of 2-hydroxynaphthalene with carbon dioxide, in which the carboxylation melt is diluted with water and dissolved therein, and the products present therein are separated and isolated, characterized in that the alkali-soluble acidic resins are separated during the work-up process of the carboxylation product by means of a cationic surface-active compound.

2. The process according to claim 1, characterized in that the cationic surface-active compound used contains a quaternary nitrogen atom.

3. The process according to claim 1, characterized in that the cationic surface-active compound used is a compound of the general formula (1)

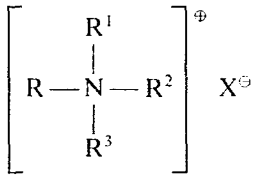

(1)

in which R, $R^1$, $R^2$ and $R^3$ are identical to or different from each other and each is an optionally substituted aliphatic radical, an aralkyl radical or an aryl radical or two or three of the aliphatic radicals may form, together with the nitrogen atom, a heterocyclic ring, and the sum of the carbon atoms of the substituents R, $R^1$, $R^2$ and $R^3$ is at least 8, and in which $X^\ominus$ is the equivalent of an inorganic or organic acid.

4. The process according to claim 3, characterized in that, in the cationic surface-active compound of the general formula (1), used, R is an alkyl radical of from 8 to 20 carbon atoms, $R^1$ is an alkyl radical of from 1 to 20 carbon atoms or is a di- to hexaethyleneglycol ether radical or is a hydroxyalkyl radical of from 2 to 6 carbon atoms or is a phenylalkyl radical of from 4 to 12 carbon atoms in the alkyl moiety, $R^2$ is an alkyl radical of from 1 to 8 carbon atoms or a hydroxyalkyl radical of from 2 to 6 carbon atoms, and $R^3$ is an alkyl radical of from 1 to 8 carbon atoms or the benzyl radical.

5. The process according to claim 1, characterized in that the cationic surface-active compound used is coconut-benzyl-dimethyl-ammonium chloride.

6. The process according to any one of claims 1 to 5, characterized in that the cationic surface-active compound is used in an amount of from 0.1 to 5 percent by weight, relative to 2-hydroxynaphthalene-3-carboxylic acid contained in the carboxylation mixture.

7. The process according to any one of claims 1 to 5, characterized in that the cationic surface-active compound is used in an amount of from 0.5 to 3.0 percent by weight, relative to 2-hydroxynaphthalene-3-carboxylic acid contained in the carboxylation mixture.

**Revendications**

1. Procédé pour isoler l'acide hydroxy-2-naphtalène-carboxylique-3 à partir du produit provenant de la réaction du sel sodique de l'hydroxy-2 naphtalène avec le dioxyde de carbone, dans lequel on dilue la masse fondue de carboxylation avec de l'eau et on l'y dissout, et on sépart les produits qui se trouvent dans la solution ainsi obtenue, procédé caractérisé en ce qu'on sépare les résines acides solubles dans les alcalis au moyen d'un composé surfactif cationique au cours du traitement complémentaire du produit de carboxylation.

2. Procédé selon la revendication 1, caractérisé en ce que le composé surfactif cationique utilisé contient un atome d'azote quaternaire.

3. Procédé selon la revendication 1, caractérisé en ce que le composé surfactif cationique utilisé est un composé répondant à la formule générale (1)

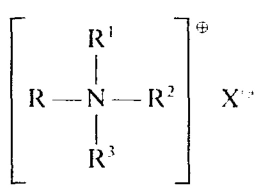

(1)

dans laquelle R, $R^1$, $R^2$ et $R^3$ sont identiques les uns aux autres ou sont différents les uns des autres et représentent chacun un radical aliphatique éventuellement substitué, un radical aralkyle ou un radical aryle, deux ou trois des radicaux aliphatiques pouvant former ensemble et avec l'atome d'azote un noyau hétérocyclique, avec la condition qu la somme des atomes de carbone des substituants R, $R^1$, $R^2$ et $R^3$ soit au moins égale à 8, et dans laquelle $X^\ominus$ représente l'équivalent d'un acide minéral ou organique.

4. Procédé selon la revendication 3, caractérisé en ce que, dans le composé surfactif cationique utilisé qui répond à la formule générale (1), R représente un radical alkyle contenant de 8 à 20 atomes de carbone, $R^1$ un radical alkyle contenant de 1 à 20 atomes de carbone ou un radical de di- à hexa-éthylène-glycol-éther ou un radical hydroxyalkyle contenant de 2 à 6 atomes de carbone ou un radical phénylalkyle contenant de 4 à 12 atomes de carbone dans la partie alkylique, $R^2$ représente un radical alkyle contenant de 1 à 8 atomes de carbone ou un radical hydroxy-alkyle contenant de 2 à 6

atomes de carbone, et $R^3$ représente un radical alkyle contenant de 1 à 8 atomes de carbone ou un radical benzyle.

5. Procédé selon la revendication 1, caractérisé en ce que le composé surfactif cationique utilisé est le chlorure d'alkyl-benzyl-diméthylammonium dont l'alkyle provient du coco.

6. Procédé selon l'une quelconque des revendications 1 à 5, caractérisé en ce que le composé cationique surfactif est utilisé en une quantité de 0,1 à 5% en poids par rapport à l'acide hydroxy-2 naphtalène-carboxylique-3 contenu dans le mélange de carboxylation.

7. Procédé selon l'une quelconque des revendications 1 à 5, caractérisé en ce que le composé cationique surfactif est utilisé en une quantité de 0,5 à 3,0% en poids par rapport à l'acide hydroxy-2 naphtalène-carboxylique-3 contenu dans le mélange de carboxylation.